# EUROPÄISCHE PATENTANMELDUNG

(11) **EP 1 836 983 A1**
(43) Veröffentlichungstag der Anmeldung: **26.09.2007**
(21) Anmeldenummer: 06111415.3
(22) Anmeldetag: 20.03.2006
(51) Int. Cl.: A61B 17/88

(54) **Instrument zum Zuführen von festen Füllmitteln in einen Knocheninnenraum**

(71) Anmelder: Sidler, Bruno, 5737 Menziken (CH)
(72) Erfinder: Sidler, Bruno, 5737 Menziken (CH)
(74) Vertreter: Dr. Graf & Partner

(57) **Zusammenfassung**

Das Instrument (1) zum Zuführen von festen Füllmitteln (2) in einen Knocheninnenraum umfasst eine Kanüle (3) sowie einen damit verbundenen Handgriff (4), wobei ein Speicher (5) verschiebbar im Handgriff (4) gelagert ist, und wobei der Speicher (5) eine Mehrzahl von Kammern (5a) zur Aufnahme von festen Füllmitteln (2) aufweist, wobei die Kammern (5a) derart im Speicher (5) angeordnet sind, dass die Kammern (5a) durch Verschieben des Speichers (5) nacheinander in eine Abgabeposition (A) bezüglich der Kanüle (3) positionierbar sind, sodass die Füllmittel (2) der sich jeweils in der Abgabeposition (A) befindlichen Kammer (5b) der Kanüle (3) zuführbar sind.

## Beschreibung

Die vorliegende Erfindung betrifft ein Instrument zum Zuführen von festen Füllmitteln in einen Knocheninnenraum gemäss dem Oberbegriff von Anspruch 1.

Das Dokument WO2005/094735, dessen Offenbarung hiermit durch Referenzierung hierin aufgenommen wird, beschreibt ein Füllmittel, eine Zuführvorrichtung sowie ein Verfahren zum Ausbilden einer Stützstruktur in einem Knocheninnenraum. Dabei wird eine Mehrzahl von Stützkörpern in einen Knocheninnenraum, insbesondere in einen Wirbelkörper eingeführt.

Es ist Aufgabe der vorliegenden Erfindung ein Instrument zum Zuführen von festen Füllmitteln in einen Knocheninnenraum vorzuschlagen, welches ein besonders einfaches, sicheres und zuverlässiges Zuführen erlaubt.

Diese Aufgabe wird gelöst mit einem Instrument aufweisend die Merkmale von Anspruch 1. Die Unteransprüche 2 bis 14 betreffen weitere, vorteilhaft ausgestaltete Instrumente.

Die Aufgabe wird insbesondere gelöst mit einem Instrument umfassend eine Kanüle sowie einen damit verbundenen Handgriff, wobei ein Speicher verschiebbar im Handgriff gelagert ist, und wobei der Speicher eine Mehrzahl von Kammern zur Aufnahme von festen Füllmitteln aufweist, wobei die Kammern derart im Speicher angeordnet sind, dass die Kammern durch Verschieben des Speichers nacheinander in eine Abgabeposition bezüglich der Kanüle positionierbar sind, sodass die Füllmittel der sich jeweils in der Abgabeposition befindlichen Kammer der Kanüle zuführbar sind.

Das erfindungsgemässe Instrument weist die Vorteile auf, dass dieses sehr einfach zu bedienen ist, dass das Instrument sehr einfach mit dem Knocheninnenraum verbindbar ist, und dass die Füllmittel sehr einfach und sicher dem Knocheninnenraum zuführbar sind. Zudem kann ein Chirurge auf einfache weise selbst bestimmen wie viele Füllmittel dem Knocheninnenraum zugeführt werden, da das Instrument einen mit Füllmitteln bestückten Speicher umfasst.

Das erfindungsgemässe Instrument ermöglicht insbesondere einen besonders einfachen Zugang zur Wirbelsäule, indem eine kurze und vor allem schlanke Kanüle verwendet wird, welche am Wirbelkörper angesetzt wird. Diese während dem Einführen üblicherweise mit einem Trokar versehene Kanüle weist den großen Vorteil, dass beim Einführen durch die empfindliche Rückenmuskulatur hindurch genau diese Muskeln maximal geschont werden. Dadurch wird der Patient nach der Operation schneller wieder beweglich. Auch ausgeprägte Narbenbildungen im Bereich der Rückenmuskeln bleiben ihm erspart.

Der Handgriff ist in einer bevorzugten Ausführungsform derart ausgestaltet, dass dieser mit der linken Hand gehalten werden kann. In einer bevorzugten Ausführungsform weist das Instrument eine Taste auf, mit welcher der Speicher bewegt werden kann, um Füllmittel in eine Abgabeposition zu positionieren. In einer bevorzugten Ausführungsform kann diese Taste mit dem Daumen der linken Hand betätigt werden. Ein Stössel wird vorzugsweise in der rechten Hand gehalten. Dieser Stössel wird in den Handgriff eingeführt, wobei mit der Stösselspitze zugleich die sich in der Abgabeposition befindlichen Füllmittel aus dem Speicher gestossen werden, und danach die Füllmittel über die Kanüle in den Knocheninnenraum gestossen werden. Das manuelle Bedienen des Stössels weist den Vorteil auf, dass die aufzuwendende Stosskraft beim Einführung der Füllmittel in den Knocheninnenraum von Chirurgen gespürt wird, sodass beispielsweise eine zu grosse Stosskraft, welche den Knochen beschädigen könnte, vermieden werden kann. Das manuelle Bedienen des Stössels weist zudem den Vorteil auf, dass die sich im Knocheninnenraum befindlichen Füllmittel positioniert werden können, beispielsweise wenn während dem Einführung der Füllmittel noch ein Bild gebendes Verfahren, beispielsweise ein Röntgengerät, verwendet wird.

Der Speicher kann auf unterschiedlichste Art ausgestaltet sein, beispielsweise auch in Form eines stangenförmigen, sich in Längsrichtung erstreckenden Speichers, oder eines kreisförmigen, als Trommel ausgestalteten Speichers.

Das Instrument wird vorzugsweise zusammen mit einem Trokar ausgeliefert, welcher insbesondere zum Eröffnen des Knocheninnenraumes dient. Bevorzugt wird das Instrument mit durch die Kanüle verlaufendem und am Handgriff befestigtem Trokar ausgeliefert, wobei zudem der Speicher bereits mit Füllmitteln gefüllt ist, sodass die wesentlichen Komponenten als eine einzige Einheit geliefert werden.

Das erfindungsgemässe Instrument ermöglicht es einem Chirurgen mit zwei Händen alle wesentlichen Verfahrensschritte ohne zusätzliche Hilfe durchzuführen, weshalb das Instrument sehr einfach und zuverlässig zu bedienen ist.

Das erfindungsgemässe Instrument ist insbesondere zum Füllen von Wirbelkörpern geeignet. Das erfindungsgemässe Instrument erlaubt jedoch jegliche Knocheninnenräume mit Füllmitteln zu versehen, beispielsweise auch auf unterschiedlichste Arten geschädigte oder geschwächte Knochen, beispielsweise durch Trauma, Infektion, Abnutzung, Tumorwachstum oder degenerative Krankheiten wie Osteoporose. Bei älteren Menschen stellt insbesondere die Osteoporose, das heisst der Abbau der Spongiosa, ein Problem dar, weil dadurch die Belastungsfähigkeit des Knochens geschwächt wird, was zur Folge hat, dass vermehrt Knochenbrüche auftreten, vor allem an der Wirbelsäule, am Schenkelhals und am Handgelenk. Das erfindungsgemässe Instrument ermöglicht in derartige Knochen Füllmittel einzuführen, wobei das Einführen, falls erforderlich, zudem ein Aufrichten eines Knochens, beispielsweise eines eingebrochenen Wirbelkörpers ermöglicht.

Die Erfindung wird nachfolgend an Hand von Figuren im Detail beschrieben, wobei dieselben Bezugszeichen dieselben Gegenstände bezeichnen. Es zeigen:
- Fig. 1: eine perspektivische Ansicht des Instruments zum Zuführen von festen Füllmitteln in einen Knocheninnenraum;
- Fig. 2: eine perspektivische Ansicht des Instruments von unten;
- Fig. 3: eine seitliche Ansicht des Instruments;
- Fig. 4: eine Ansicht der Stirnseite des Instruments;
- Fig. 5: eine Innenansicht der unteren Hälfte des Handgriffteils des Instruments gemäss Figur 1;
- Fig. 6: eine Detailansicht des linken Endabschnittes der oberen Hälfte des Handgriffteils;
- Fig. 7: eine Draufsicht auf einen Speicher;
- Fig. 8: eine Seitenansicht des Speichers gemäss Figur 7;
- Fig. 9a: eine Draufsicht auf ein Füllmittel;
- Fig. 9a: eine Seitenansicht einer Mehrzahl gestapelter Füllmittel;
- Fig. 10, 11: Ansichten eines Trokar;

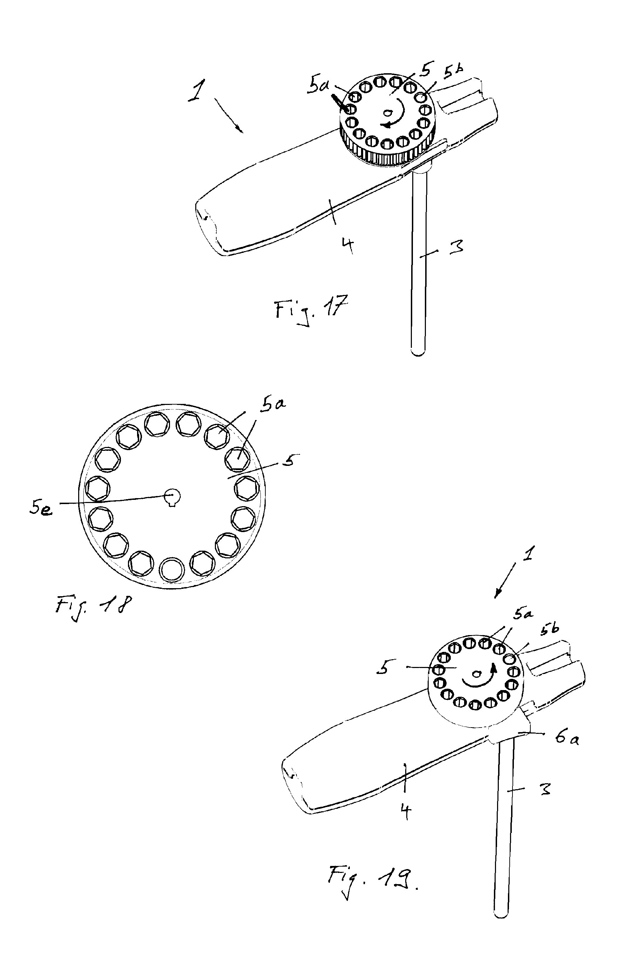

- Fig. 12: ein Stössel;
- Fig. 13: ein Instrument während dem Verbinden mit einem Knochen;
- Fig. 14: einen Schnitt durch einen Wirbelknochen während dem Einführen der Füllmittel;
- Fig. 15a-15e: eine Mehrzahl unterschiedlich ausgestalteter Stösselspitzen;
- Fig. 16: das Instrument in Kombination mit einem aktiv angetriebenen Stössel,
- F. 17: eine perspektivische Ansicht eines weiteren Ausführungsbeispiels eines Instruments mit drehbarem Speicher;
- Fig. 18: eine perspektivische Ansicht eines drehbaren Speichers;
- Fig. 19: eine perspektivische Ansicht eines weiteren Ausführungsbeispiels eines Instruments mit drehbarem Speicher.

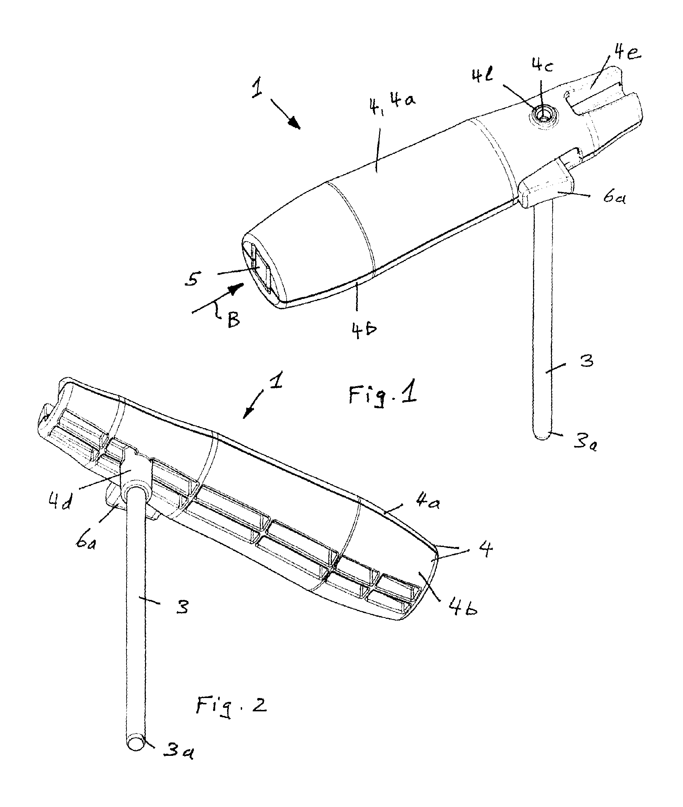

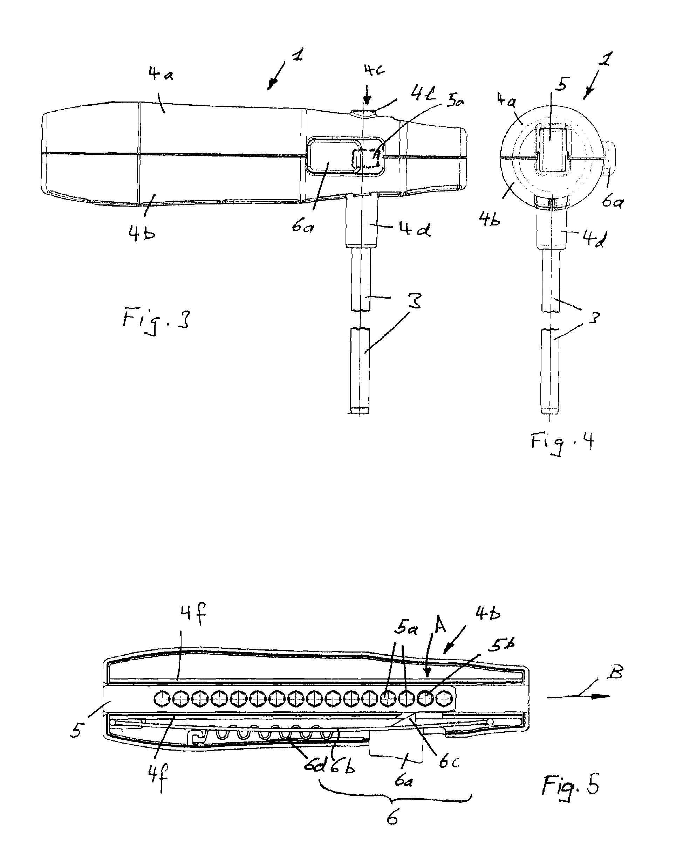

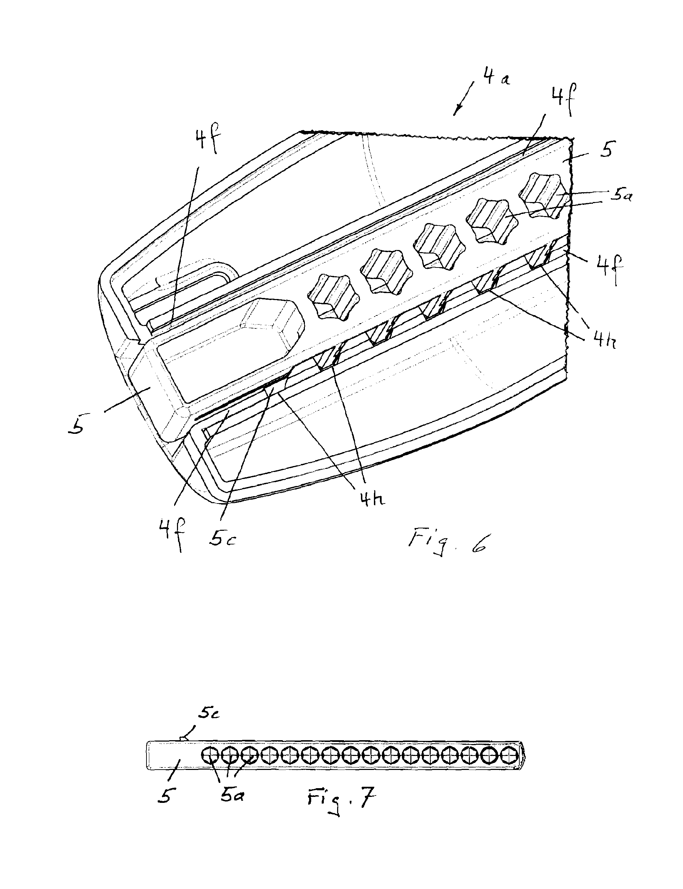

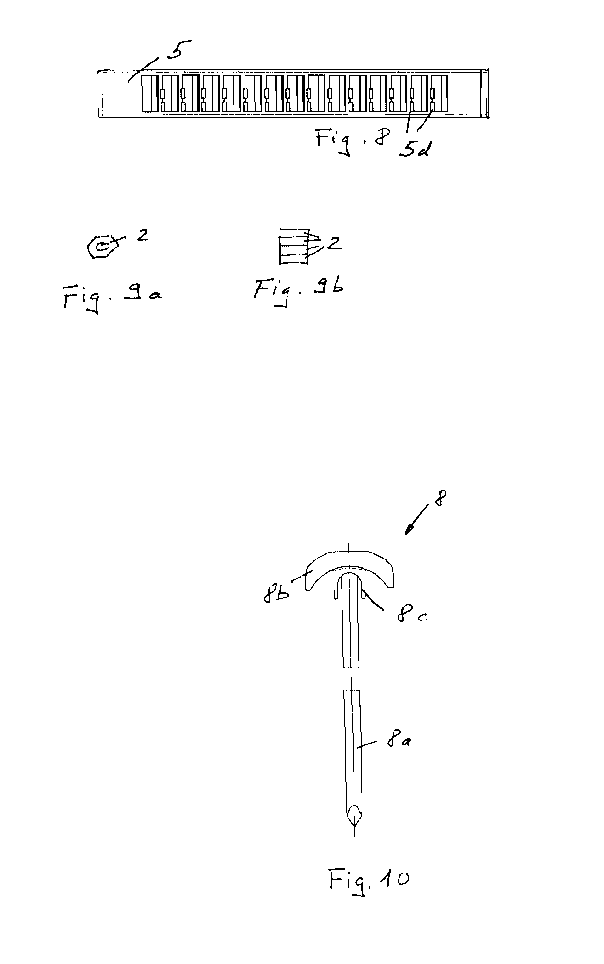

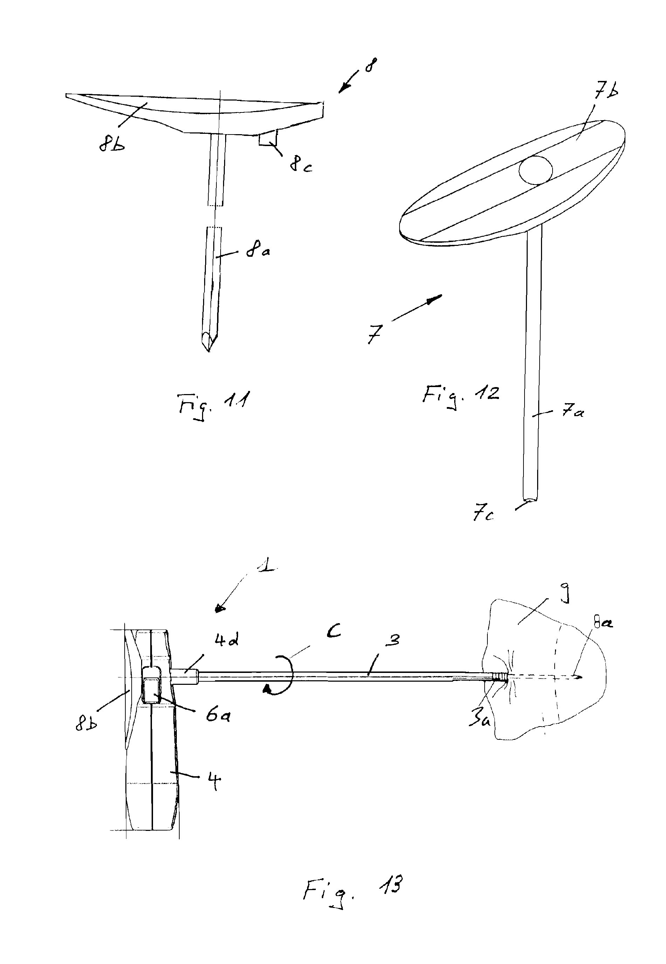

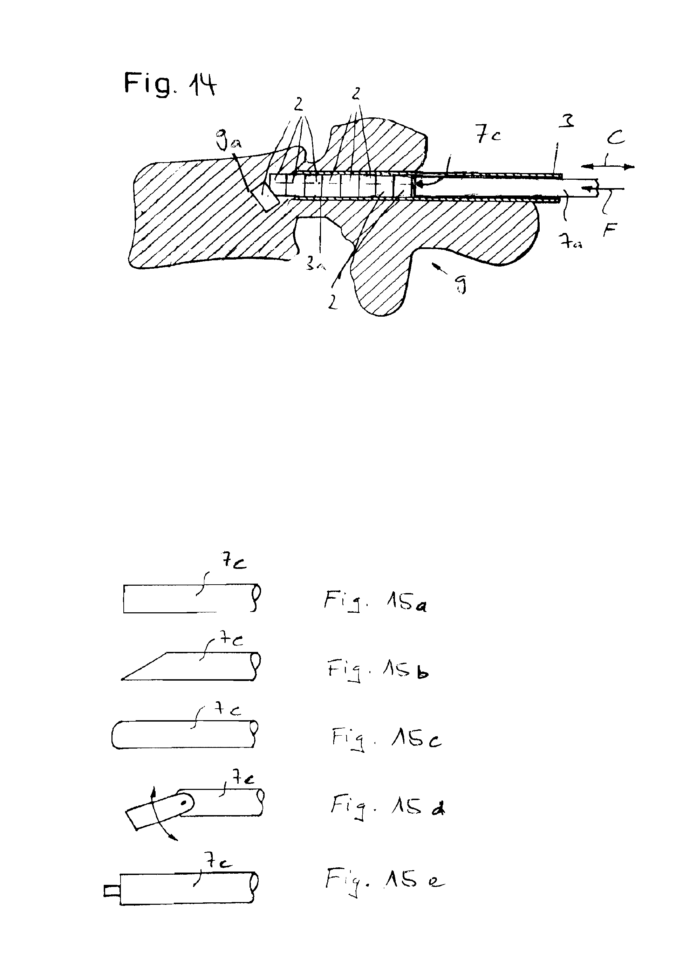

Die Figur 1 bis 4 zeigen dasselbe Instrument 1 zum Zuführen von festen Füllmitteln in einen Knocheninnenraum aus unterschiedlichen Blickrichtungen. Das Instrument 1 umfasst einen Handgriff 4 sowie eine Kanüle 3, welche fest miteinander verbunden sind. Der Handgriff 4 umfasst eine obere Gehäusehälfte 4a sowie eine untere Gehäusehälfte 4b, welche ebenfalls fest miteinander verbunden sind. Der Handgriff 4 umfasst zudem eine Halterung 4d für die Kanüle 3 sowie eine Zugangsöffnung 4c, welche von einer vorstehenden Hülse 41 umgeben ist. Wie aus der Seitenansicht gemäss Figur 3 ersichtlich, ist die Zugangsöffnung 4c in geradliniger Verlängerung zur Kanüle 3 angeordnet, sodass der Schaft 8a,7a eines Trokars 8 oder eines Stössels 7 in die Zugangsöffnung 4c einführbar ist, und über die Kanüle 3 bis zur Kanülenspitze 3a, und falls erforderlich auch darüber hinaus eingeführt werden kann.

Im dargestellten Ausführungsbeispiel ist im Innern des Handgriffs 4, in dessen Längsrichtung verlaufend, ein in Bewegungsrichtung B verschiebbarer Speicher 5 gelagert. Der Handgriff 4 umfasst zudem ein Vorschubteil 6 mit einem Taster 6a, durch dessen Betätigen der Speicher 5 um eine definierte Wegstrecke in Bewegungsrichtung B verschiebbar ist.

Das dargestellte Instrument 1 ist für die linke Hand optimiert, indem der Handgriff 4 mit der linken Hand umschlossen werden kann, derart, dass die Taste 6a mit dem Daumen in Verschiebungsrichtung B betätigt werden kann. Somit kann der Handgriff 4 sowohl gehalten werden, als auch die Lage des Speichers 5 verändert werden.

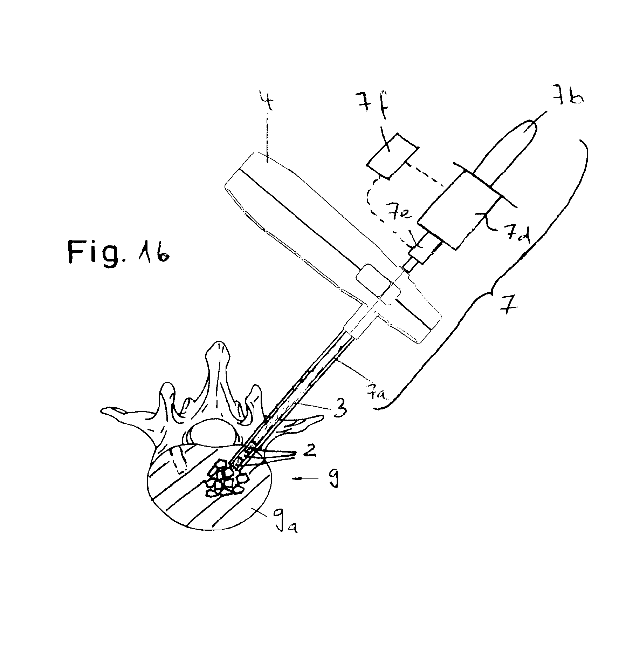

Figur 5 zeigt eine Innenansicht der unteren Hälfte 4b des Handgriffs 4. Die untere Handgriffhälfte 4b weist in Verlaufsrichtung B verlaufende seitliche Führungen 4f auf, zwischen welchen ein Speicher 5 in Richtung B verschiebbar gelagert ist. Der Speicher 5 umfasst eine Mehrzahl von in Richtung B beabstandet angeordneter Kammern 5a, 5b. Eine der Kammern, mit 5b bezeichnet, befindet sich direkt über der Kanüle 3. Diese Position einer Kammer wird als Abgabeposition A bezeichnet, da sich die Kammer 5b unmittelbar über der Kanüle 3 befindet. Das Instrument 1 umfasst zudem ein Vorschubteil 6, welches im dargestellten Ausführungsbeispiel eine Taste 6a, eine Feder 6b und eine Eingriffnase 6c sowie eine Rückstellfeder 6d umfasst.

Figur 8 zeigt eine Seitenansicht des Speichers 5 mit einer Mehrzahl von in Längsrichtung B beabstandeten Nuten 5d. Diese Nuten 5d sind in der in Figur 5 dargestellten Anordnung zur Eingriffnase 6c hin ausgerichtet, so dass beim Betätigen der Taste 6a deren Eingriffnase 6c in die Nut 5b eingreift, wobei die Taste 6a zugleich in Richtung B bewegt wird, sodass der Speicher 5 um eine derart definierte Wegstrecke in Richtung B verschoben wird, dass die zur Kammer 5b nächstfolgende Kammer 5a in die Abgabeposition A gelangt.

Figur 7 zeigt eine Draufsicht auf den Speicher 5 mit einer Mehrzahl von in Verlaufsrichtung beabstandet angeordneten Kammern 5a sowie mit einem vorstehenden Vorsprung 5c.

Figur 6 zeigt eine Detailansicht der oberen Hälfte 4a des Handgriffs 4. Die unten dargestellte seitliche Führung 4f weist regelmässig beabstandete Nuten 4h auf, in welche der Vorsprung 5c des Speichers 5 eingreift, so dass der Speicher 5 in Verschiebungsrichtung B bezüglich dem Handgriff 4 definierte Einrastpositionen aufweist. Diese Einrastpositionen sind derart gewählt, das immer eine Kammer 5a in die Abgabeposition A zu liegen kommt in welcher die sich in der Kammer 5a befindlichen Füllmittel 2 der Kanüle 3 zuführbar sind.

Figur 9a zeigt ein Füllmittel 2 in einer Aufsicht. Das Füllmittel ist im dargestellten Ausführungsbeispiel als sechskantiger Körper mit einem zylinderförmigen Innenraum ausgestaltet. Figur 9b zeigt vier übereinander gestapelte Füllmittel 2. In der Darstellung gemäss Figur 5 sind üblicherweise zumindest einige der Kammern 5a und vorzugsweise alle Kammern 5a mit den in Figur 7a und 7b dargestellten Füllmitteln 2 gefüllt.

Die sich in der Kammer 5b befindlichen Füllmittel 2 können, beispielsweise über ein senkrecht zur Betrachtungsebene wirkenden Stössel 7, nach unten in die sich darunter befindliche Kanüle 3 gestossen werden.

Der in den Figuren 7 und 8 dargestellte Speicher 5 weist vorzugsweise zwischen 4 und 20 in Längsrichtung beabstandete Kammern 5a auf. In jeder Kammer 5a können, abhängig von der Höhe des verwendeten Füllmittels 2 zwischen 1 und 20 einzelne wie in Figur 9a dargestellte Füllmittel 2 angeordnet sein. In einer bevorzugten Ausgestaltung ist der Innenquerschnitt der Kammer 5a entsprechend der Aussenkontur der Füllmittel 2 ausgestaltet. Im dargestellten Ausführungsbeispiel ist die Aussenkontur des Füllmittels 2 sechskantig ausgestaltet, und die Innenkontur der Kammer 5a entsprechend ebenfalls sechskantig ausgestaltet. Vorzugsweise werden alle Kammern 5a mit Füllmitteln 2 derselben Grösse gefüllt. Es ist jedoch auch möglich die Kammern 5a mit Füllmitteln 2 unterschiedlicher Grösse, das heisst einem unterschiedlichen Durchmesser, einer unterschiedlichen Aussenkontur und/oder auch einer unterschiedlichen Höhe zu füllen. Dazu kann es sich als vorteilhaft erweisen auch den Innenquerschnitt der Kammer 5a entsprechend der jeweiligen Aussenkontur des Füllmittels 2 anzupassen. Die Füllmittel 2 können in einer Vielzahl möglicher Aussenkonturen ausgestaltet sein, beispielsweise auch kreisförmig, elliptisch, quadratisch, rechteckig usw.

Figur 10 zeigt eine Frontansicht und Figur 11 eine Seitenansicht eines Trokars 8, welcher einen Schaft 8a mit einer scharfen, dreikantigen Spitze und einen Betätigungshandgriff 8b umfasst. Der Trokar 8 umfasst zudem ein Befestigungsmittel 8c, mit Hilfe welchem der Trokar 8 über die Ausnehmung 4e am Handgriff 4 befestigbar ist.

Figur 12 zeigt einen Stössel 7, umfassend einen Handgriff 7b sowie einen Schaft 7a mit Stösselende 7c.

Das gesamte Instrument 1 wird vorzugsweise in der in Figur 13 dargestellten Anordnung ausgeliefert, indem der Trockar 8 mit Hilfe des Befestigungsmittels 8c am Handgriff 4 befestigt ist, und indem der Schaft 8a durch die Zugangsöffnung 4c, die zylinderförmige Kammer 5b und die Kanüle 3 verläuft, sodass die Spitze des Schaftes 8a vorne aus der Kanülenspitze 3a vorsteht. Diese Anordnung weist den zusätzlichen Vorteil auf, dass sich der Speicher 5, auf Grund des durch die Kammer 5b verlaufenden Schaftes 8a, nicht in Bewegungsrichtung B verschieben kann, und somit in einer definierten Lage blockiert und gehalten ist. Erst nach dem vollständigen Entfernen des Trokar 8 aus dem Instrument 1 kann der Speicher 5 bezüglich des Handgriffs 4 verschoben werden.

Wie in Figur 13 dargestellt kann das Instrument 1 beispielsweise nun derart in den menschlichen Körper eingeführt werden, dass zuerst die Spitze des Trokar 8 eingeführt wird. Im dargestellten Beispiel wird der Trokar 8 in einen nur schematisch dargestellten Wirbelkörper 9 eingeführt. Danach wird die Kanülenspitze 3a auf den Wirbelkörper 9 aufgesetzt, und die Kanüle 3 durch Drehen am Handgriff 4 in Drehrichtung C in den Wirbelkörper 9 geschraubt. Der Handgriff 4 ist vorzugsweise derart ausgestaltet, dass der Handgriff 4 während dieser Schraubbewegung auch mit der rechten Hand oder mit beiden Händen betätigt werden kann. Sobald die Kanüle 3 genügend tief im Wirbelkörper 9 angeordnet ist wird der Trokar 8 vom Handgriff 4 gelöst und vollständig aus dem Instrument 1 entfernt. Somit wird der Speicher 5 im Handgriff 4 deblockiert und dadurch verschiebbar. Der Handgriff 4 kann nun vorteilhafterweise mit der linken Hand gehalten werden. Durch ein Betätigen der Taste 6a wird die erste, der Kammer 5b nachfolgend angeordnete Kammer 5a in die Abgabeposition A geschoben. Daraufhin wir mit der rechten Hand der Stössel 7 über die Zugangsöffnung 4c in den Handgriff 4 eingeführt und die sich in der Kammer 5a befindlichen Füllmittel 2 durch den Stössel 7 in die Kanüle 3 geschoben. Figur 3 zeigt schematisch die sich in der Abgabeposition A befindliche Kammer 5a mit Füllmitteln 2, welche durch den Stössel 7 in Richtung der Kanüle 3 geschoben werden.

Figur 14 zeigt schematisch einen Schnitt durch einen Wirbelkörper 9, in dessen Innenraum 9a die Füllmittel 2 geschoben werden, indem der Stössel 7 über das Stösselende 7c eine Kraft F auf die sich in der Kanüle 3 befindlichen Füllmittel 2 ausübt. Die Anzahl der gleichzeitig zugeführten Füllmittel 2 kann dadurch bestimmt werden, dass entweder jeweils alle sich in einer Kammer 5a befindlichen Füllmittel 2 in den Wirbelkörper 9 geschoben werden, oder dass nach dem ersten leeren einer Kammer 5a der Stössel 7 zurückgezogen wird, die Taste 6a betätigt wird, und der Inhalt der nachfolgenden Kammer 5a ebenfalls durch den Stössel 7 in die Kanüle 3 geschoben wird. Dieser Vorgang kann, falls erwünscht, mehrmals wiederholt werden, um beispielsweise 2, 3, 5 oder 10 Inhalte der Kammern 5a vorerst in die Kanüle 3 zu verschieben, bevor alle derart in der Kanüle 3 nacheinander angeordneten Füllmittel 2 mit Hilfe des Stössels 7 in den Wirbelkörper 9 gedrückt werden.

Es kann sich als Vorteilhaft erweisen eine Mehrzahl von Stösseln 7 mit, wie in den Figuren 15a bis 15e dargestellt, unterschiedlich ausgestalteten Spitzen 7c vorzusehen. Nachdem die Füllmittel 2 im Wirbelkörper 9 eingeführt sind kann deren Lage teilweise noch beeinflusst werden, indem ein Stössel 7 mit einer entsprechend geformten Spitze 7c verwendet wird, um, beispielsweise mit der in Figur 15b dargestellten Spitze 7c, die Füllmittel 2 seitlich zu verschieben. Die Lage der Füllmittel 2 im Wirbelkörper 9 wird vorzugsweise mit Hilfe eines Röntgengerätes sichtbar gemacht.

Figur 16 zeigt schematisch ein weiteres Ausführungsbeispiel eines Stössels 7, mit dessen Hilfe Füllmittel 2 über ein Instrument 1 mit Kanüle 3 einem Wirbelkörper 9 zugeführt werden. Der dargestellte Stössel 7 umfasst einen Handgriff 7b, einen aktiven Antrieb 7d, einen Sensor 7e, eine Regelungsvorrichtung 7f und einen Schaft 7a. Der Antrieb ist beispielsweise als elektromotorischer Antrieb oder als Druckluftantrieb ausgestaltet, und ermöglicht vorzugsweise auch eine Vibrationsbewegung zu erzeugen, um beispielsweise die sich im Wirbelkörper 9 befindlichen Füllmittel 2 in verdichteter Form einzulagern. Die Regelungsvorrichtung 7f erlaubt beispielsweise mit dem Sensor 7e Kraft oder Weg zu messen, und die maximale Schubkraft oder den maximalen Hub der Vibrationsbewegung zu begrenzen.

Die Kanüle 3 kann auch, insbesondere falls keine Schraubbewegung zum Befestigen am Knochen erforderlich ist, einen gekrümmten Verlauf aufweisen. Die Kanüle 3 ist vorzugsweise aus Metall gefertigt, kann jedoch auch aus einem Kunststoff gefertigt sein. Der Stössel 7 kann aus Metall oder auch aus einem Kunststoff gefertigt sein, insbesondere auch aus einem elastischen Kunststoff.

Der Speicher 5 kann auf unterschiedliche Weise ausgestaltet sein, beispielsweise auch als Trommel mit bezüglich einer Drehachse kreisförmig in Umfangsrichtung beabstandet angeordneten Kammern 5a, wobei der Speicher drehbar im Handgriff 4 gelagert ist.

Die Füllmittel 2 können eine Vielzahl möglicher Formen aufweisen, derart, dass die Füllmittel 2 im Speicher 5 gespeichert werden können. Die Füllmittel 2 könnten beispielsweise auch kugelförmig ausgestaltet sein.

Figur 17 zeigt ein Instrument 1 mit Kanüle 3, Handgriff 4 und einem auf dem Handgriff 4 in der angedeuteten Drehrichtung drehbar gelagerten, trommelförmigen Speicher 5 mit einer sich in Abgabeposition 5b befindlichen Kammer und einer Mehrzahl weiterer in Umfangsrichtung beabstandet angeordneter Kammern 5a zur Aufnahme von Füllmitteln 2. Der Speicher 5 weist an der zylinderförmigen Aussenumfangsfläche Rillen auf, über welche die Position des Speichers 5 von Hand gedreht werden kann. Das Instrument 1 weist vorzugsweise eine Einrastvorrichtung auf, damit die Kammern 5a sich nach einem Drehen jeweils in der Abgabeposition 5b befinden.

Figur 18 zeigt eine Draufsicht auf den in Figur 17 dargestellten Speicher 5. Der Speicher ist um eine Drehachse 5e drehbar gelagert.

Figur 19 zeigt ein weiteres Ausführungsbeispiel eines Instruments 1. Im Unterschied zu der in Figur 18 dargestellten Ausführungsform weist dieses Instrument 1 einen Taster 6a auf, wobei der Taster 6a sowie der Speicher 5 derart ausgestaltet sind, dass beim Betätigen des Taster 6a der Speicher 5 um jeweils eine Position in eingezeichneter Richtung gedreht wird, sodass nacheinander folgende Kammern 5a in die Abgabeposition 5b befördert werden, sodass die sich in der Kammer 5a befindlichen Füllmittel 2 mit dem Stössel 7 der Kanüle 3 zuführbar sind.

## Patentansprüche

1. Instrument (1) zum Zuführen von festen Füllmitteln (2) in einen Knocheninnenraum, umfassend eine Kanüle (3) sowie einen damit verbundenen Handgriff (4), wobei ein Speicher (5) verschiebbar im Handgriff (4) gelagert ist, und wobei der Speicher (5) eine Mehrzahl von Kammern (5a) zur Aufnahme von festen Füllmitteln (2) aufweist, wobei die Kammern (5a) derart im Speicher (5) angeordnet sind, dass die Kammern (5a) durch Verschieben des Speichers (5) nacheinander in eine Abgabeposition (A) bezüglich der Kanüle (3) positionierbar sind, sodass die Füllmittel (2) der sich jeweils in der Abgabeposition (A) befindlichen Kammer (5b) der Kanüle (3) zuführbar sind.

2. Instrument nach Anspruch 1, **dadurch gekennzeichnet, dass** der Handgriff (4) in Verlängerungsrichtung der Kanüle (3) eine Zugangsöffnung (4c) aufweist, und dass sich die Abgabeposition (A) zwischen Kanüle (3) und Zugangsöffnung (4c) befindet.

3. Instrument nach einem der vorhergehenden Ansprüche, **dadurch gekennzeichnet, dass** der Speicher (5) stangenförmig ausgestaltet ist, mit einer Mehrzahl von in dessen Verlaufsrichtung beabstandeten Kammern (5a).

4. Instrument nach Anspruch 3, **dadurch gekennzeichnet, dass** der Speicher (5) innerhalb des Handgriffs (4) und in dessen Längsrichtung verlaufend angeordnet ist.

5. Instrument nach einem der Ansprüche 1 oder 2, **dadurch gekennzeichnet, dass** der Speicher (5) als Trommel mit kreisförmig angeordneten Kammern (5a) ausgestaltet ist.

6. Instrument nach einem der vorhergehenden Ansprüche, **dadurch gekennzeichnet, dass** ein Vorschubteil (6) den Speicher (5) derart verschiebt, dass nach dem Betätigen des Vorschubteil (6) jeweils eine Füllmittel (2) enthaltende Kammer (5b) in der Abgabeposition (A) angeordnet ist.

7. Instrument nach Anspruch 6, **dadurch gekennzeichnet, dass** das Vorschubteil (6) eine Taste (6a) umfasst, welche derart bezüglich dem Handgriff (4) angeordnet ist, dass, wenn eine Hand den Handgriff (4) umschliesst, die Taste (6a) mit dem Daumen der Hand betätigbar ist.

8. Instrument nach einem der Ansprüche 2 bis 7, **dadurch gekennzeichnet, dass** ein Stössel (7) derart ausgestaltet ist, dass dieser über die Zugangsöffnung (4c) in die Kanüle (3) einführbar ist, sodass dieser eine Stosskraft auf in der Abgabeposition (A) oder in der Kanüle (3) befindliche Füllmittel (2) ausüben kann.

9. Instrument nach einem der vorhergehenden Ansprüche, **dadurch gekennzeichnet, dass** dieses einen Trokar (8) mit einem Schaft (8a) umfasst, wobei der Schaft (8a) derart ausgestaltet ist, dass dieser über die Zugangsöffnung (4c) in die Kanüle (3) zuführbar ist.

10. Instrument nach Anspruch 9, **dadurch gekennzeichnet, dass** der Trokar (8) lösbar mit dem Handgriff (4) verbunden ist, und dass der Schaft (8a) des Trokar (8) durch die Zugangsöffnung(4c), eine leere Kammer (5a) des Speichers (5) sowie die Kanüle (3) verläuft.

11. Instrument nach einem der Ansprüche 9 oder 10, **dadurch gekennzeichnet, dass** der Trokar (8) über ein Befestigungsmittel (8c) lösbar mit dem Handgriff (4) verbunden ist.

12. Instrument nach einem der vorhergehenden Ansprüche, **dadurch gekennzeichnet, dass** die Kammern (5a) Füllmittel (2) enthalten.

13. Instrument nach Anspruch 12, **dadurch gekennzeichnet, dass** die Füllmittel (2) eine kantige, insbesondere eine sechskantige Aussenkontur aufweisen, und dass die Kammern (5a) einen der Aussenkontur des Füllmittels (2) angepassten Innenquerschnitt aufweisen.

14. Instrument nach Anspruch 12 oder 13, **dadurch gekennzeichnet, dass** jede Kammer (5a) zwischen 1 und 20 Füllmittel (2) enthält, und dass jeder Speicher (5) zwischen 4 und 20 Kammern (5a) aufweist.

15. Speicher (5) für ein Instrument (1) nach einem der vorhergehenden Ansprüche, umfassend eine Mehrzahl von mit Füllmitteln (2) gefüllte Kammern (5a).
